# EUROPEAN PATENT APPLICATION

(11) **EP 1 607 382 A1**
(43) Date of publication of application: **21.12.2005**
(21) Application number: 04014231.7
(22) Date of filing: 17.06.2004
(51) Int. Cl.: C07C 43/11, C07C 41/38, D06L 1/00

(54) **Method for producing narrow range alcoxylates**

(71) Applicant: Linde Aktiengesellschaft, 65189 Wiesbaden (DE)
(72) Inventor: Karthäuser, Joachim, 19251 Sollentuna (SE)
(74) Representative: Gellner, Bernd

(57) **Abstract**

The invention relates to a process for separating a composition into at least two fractions. Said composition comprises a mixture of organic alcohols in the carbon number range 4 to 18 which previously has been subjected to reaction with organic epoxides, including ethylene oxide, propylene oxide or butylenes oxide, or mixtures thereof, simultaneously or sequentially, said composition therefore containing a broad distribution of epoxide or alcoxy units. Pressurized carbon dioxide in at least 90% concentration by weight is used as separation agent and said composition is separated in at least two fractions which differ in the average number of alcoxy units which are attached to said organic alcohols, and may differ in the average molecular weight of said organic alcohols.

## Description

The invention relates to a process for separating a composition into at least two fractions whereby said composition comprises a mixture of organic alcohols in the carbon number range 4 to18 which previously has been subjected to reaction with organic epoxides, including ethylene oxide, propylene oxide or butylenes oxide, or mixtures thereof, simultaneously or sequentially, said composition therefore containing a broad distribution of epoxide or alcoxy units.

Non-ionic surfactants are made by alcoxylation of alcohols, e.g. by base catalysed addition of ethylene oxide (EO) or propylene oxide (PO) to organic straight chain alcohols with 6 to 18 carbon atoms. These nonionic surfactants are mixtures of alcohols with a broad (Poisson-type) distribution of 0, 1, 2, 3, 4, 5 and so forth alcoxy units.

In terms of washing performance, certain fractions are most desired, often those fractions where the hydrophobic portion (the alkyl chain) and the hydrophilic portion (the alcoxy chain) are in a certain balance. Alcohols with low alcoxylation degree are not water-soluble, alcohols with high alcoxylation degree are too watersoluble to act as washing agent.

It is, therefore, desirable to offer alcoxylates with a narrow distribution of alcoxy groups.

Separations of liquids (olive oil, waxes etc.) using supercritical CO2 is well known.

Separation of nonionic surfactants into fractions of low and high alcoxy content using solvents is not practiced industrially due to solvent handling issues and recovery costs. Instead, the approach in the industry has been to develop catalysts which enhance or favour the production of more narrow distributions.

The following abbreviations are used:
RO = alcohol, e.g. fatty or synthetic alcohol in the carbon number range 1-20
EO = ethylene oxide unit
PO = propylene oxide unit
BO = butylenes oxide unit
(EO) 3 = three EO units chemically coupled
The term Ethoxylate is conveniently used in this text to denote all types of glycols and other alcoxylates.

Nonionic surfactants are commercially available as broad distributions, comprising RO-(EO) x molecules with x being 0, 1, 2 and so forth. However, for a given process, e.g. washing in aqueous or carbon dioxide based systems, or as anti-freeze fluid or additive in textile processing or in the oil industry as drilling mud and in other applications, very often a specific ethoxylation degree and, in other words, a specific hydrophilic/lipophilic balance (HLB value) is desired.

Therefore, the problem with the prior art is that conventional production processes offer the desired molecules only as an average, and the desired product is diluted in significant amounts of material which either is too hydrophilic or too lipophilic for the desired function.

In addition, free alcohol and species with low alcoxylation degree create adverse smell in washing formulations, which is another incentive to minimize their concentrations. These species can easily be recycled into the alcoxylation reactors.

Approaches to improve the product are the following: on laboratory scale, nonionics can be purified using various separation techniques, such as chromatography. Industrially, solvent extraction could be applied, however, handling and recovering solvents from foaming surfactants is expensive. Therefore, alcoxylate producers concentrate their efforts on developing catalysts for alcoxylation which favour narrow distributions of alcoxy units.

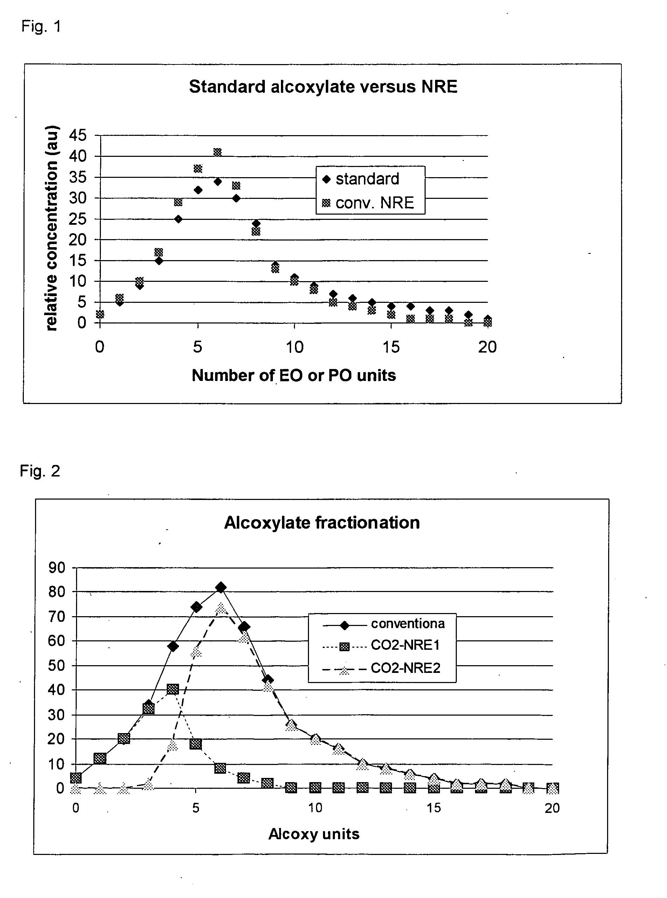

Figure 1 shows typical distributions of standard ethoxylates versus narrow range ethoxylates. As can be seen, whilst the peak of the distribution is more pronounced for NRE, the product still is a quite broad distribution. Figure 1 is an illustrative representation of the composition of industrially produced alcoxylates. For both grades, the peak of the distribution is at ca. 6 EO or PO units. The composition also contains free alcohol (zero EO units) and highly alcoxylated species with more than 10 EO units.

The former (free alcohol) is hardly water-soluble, the latter species are hardly fat-soluble. Neither are good surfactants in a situation where the HLB value of RO-(EO)6 is specified.

It is an object of the invention to improve the separation of nonionic surfactants into fractions of low and high alcoxy content.

Accordingly, the present invention concerns a process for separating a composition into at least two fractions whereby said composition comprises a mixture of organic alcohols in the carbon number range 4 to 18 which previously has been subjected to reaction with organic epoxides, including ethylene oxide, propylene oxide or butylenes oxide, or mixtures thereof, simultaneously or sequentially, said composition therefore containing a broad distribution of epoxide or alcoxy units, wherein pressurized carbon dioxide in at least 90% concentration by weight is used as separation agent and said composition is separated in at least two fractions which differ in the average number of alcoxy units which are attached to said organic alcohols, and may differ in the average molecular weight of said organic alcohols.

According to the invention a composition of nonionic surfactants, as shown principally in figure 1, is subjected to an extraction with pressurized carbon dioxide. The solvent CO2 dissolves preferentially CO2-philic species, and does not dissolve species which exhibit low solubility in CO2. Therefore, by choosing temperature, pressure, time, and volume of solvent, a given composition can be split into two fractions of low respectively high content of alcoxy groups.

Technically, ethoxylates and propoxylates behave differently as propoxylates exhibit good CO2 solubility, whereas ethoxylates are poorly soluble in CO2. Therefore, CO2 will preferentially dissolve species with high PO content, and the effect is the separation of a composition as schematically depicted in figure 2 into two fractions, see also figure 2. In the case of ethoxylates, the solubility of the alcohol moiety and the total chain length of the molecules are both significant factors, therefore the process results in the extraction of free alcohol and species with low EO content.

Suitable apparatuses for the extraction process are high-pressure counter-flow liquid-liquid extraction columns, as available from companies such as Natex of Austria. Ideally, the solvent CO2 is recycled from the dissolved fraction.

CO2 should be understood as a high pressure gas composition comprising mainly CO2. As the case may be, additives such as other gases (paraffins such as C3 to C5) or liquids (alcohols or suitable cosolvents which easily can be recovered from the product) may be employed. High Pressure should be understood as a pressure being higher than 10 bar, and liquid as well as supercritical carbon dioxide are included. The extraction temperature is case-dependant, but will be in the range of 10 up to 100 °C.

For economic reasons, the process is ideally continuous. The process may be carried out more than once, e.g. using a separated fraction, in order to further fractionate the product.

Figure 2 shows the fractionation of a conventional alcoxylate composition into two fractions. The integrals under the curves indicating the fractions add up to the original composition. The exact shape of the distributions of the fractions is a function of the process parameters, such as time, solvent volume, temperature, pressure and the like.

The invention describes a very facile and economic way to separate a conventionally produced alcoxylate mixture into at least two fractions with a low and a high average content of alcoxy groups. The invention uses dense phase carbon dioxide, liquid or supercritical CO2, as selective solvent. The invention allows an easy recovery of the solvent.

In general, narrow distributions of alcoxylates meet a given desired performance specification, typically expressed as target HLB value, much better than a broad distribution. As alcoxylates are sold as many grades, e.g. C12-C15 alcohol with an average of 3, 5, 7 and 12 EO or PO units, a broad distribution with an average of e.g. 6 EO units can be split using the invention into one fraction peaking at 4 EO units, and another fraction peaking at 7 EO units, as schematically shown in figure 2. The resulting fractions are less disperse, i.e. more active in the desired application.

The invention can furthermore be used to separate the generally unwanted part of free alcohol or alcohols with one or two alcoxy units. Said fraction can be recycled to alcoxylation or used for other purposes, e.g sulfonation.

Narrow range alcoxylates are desired compositions for cleaning also in non-aqueous media, such as textile cleaning and cleaning of industrial parts using liquid or supercritical CO2. The invention claims, apart from the process of separating alcoxylate streams into fractions, also the use of the fractions obtained as washing agents in both aqueous and non-aqueous cleaning, in particular cleaning and conditioning using dense phase CO2. Regarding cleaning, an advantage of the invention is that surfactants with good CO2 solubility may be chosen. This will avoid the deposition of surfactants on the large total surface area provided by a batch of textiles, and in particular the deposition of unpleasantly smelling agents such as free alcohols is avoided. For the CO2-based process of textile conditioning where deposition is the main purpose of the process, it is an even greater advantage that non-smelling agents are not present and therefore cannot precipitate on the textile.

A stream of nonionics, e.g. fatty alcohols in the C8-C18 range containing 1-20 alcoxy units is led counter-current to a stream of pressurised carbon dioxide, preferably supercritical CO2 (sc-CO2). CO2 dissolves preferably alcoxylates containing much PO, alternatively containing little EO. The CO2 stream enriched with certain alcoxylates is led to a distillation tank from where CO2 is recovered by distillation, and the dissolved alcoxylates are recovered. The nonionics stream less the alcoxylates which were dissolved by CO2 is led to a storage tank from which a remainder of CO2 is recovered by pressure reduction, thereafter the nonionics stream is stored separately.

The preferred mode of operation is continuous in a falling or wiped film extractor.

Preferred pressure and temperature ranges are 30-300 bar and 10-90 degree Celsius.

Within the field of detergents, it is conceivable that the concept may be used for a range of other surfactants, such alkylpolyglucosides, nonylphenol-based surfactants, fatty acid derivatives and the like..

## Claims

1. A process for separating a composition into at least two fractions whereby said composition comprises a mixture of organic alcohols in the carbon number range 4 to18 which previously has been subjected to reaction with organic epoxides, including ethylene oxide, propylene oxide or butylenes oxide, or mixtures thereof, simultaneously or sequentially, said composition therefore containing a broad distribution of epoxide or alcoxy units, **characterized in that** pressurized carbon dioxide in at least 90% concentration by weight is used as separation agent and said composition is separated in at least two fractions which differ in the average number of alcoxy units which are attached to said organic alcohols, and may differ in the average molecular weight of said organic alcohols.

2. Use of said fraction obtained according to the process according to claim 1 as improved surfactant, or as feedstock for improved surfactants.

3. Use of said fraction according to claim 2 in aqueous cleaning processes.

4. Use of said fraction according to claim 2 in cleaning processes using liquid and supercritical CO2.

5. Use of said fraction according to claim 2 in cleaning processes using organic solvents.

6. Use of said fraction of alcoxylates obtained by the process according to claim 1, and containing a low number of alcoxy units including free alcohol, as raw material for alcoxylation, said process resulting in surfactants with a low concentration of free organic alcohol.
